# EUROPEAN PATENT SPECIFICATION

(11) **EP 2 371 333 B1**
(45) Date of publication and mention of the grant of the patent: **22.04.2015**
(21) Application number: 09835120.8
(22) Date of filing: 24.12.2009
(51) Int. Cl.: A61F 13/15, A61F 13/49, A61F 13/53, A61F 13/539, A61F 13/475, A61F 13/47, A61F 13/533, A61F 13/551

(54) **THIN ABSORPTIVE ARTICLE**
DÜNNER SAUGFÄHIGER ARTIKEL
ARTICLE ABSORBANT MINCE

(30) Priority: 25.12.2008 JP 2008331035
(43) Date of publication of application: 05.10.2011
(73) Proprietor: Unicharm Corporation, Ehime 799-0111 (JP)
(72) Inventor: KUDO, Jun, Kanonji-shi Kagawa 769-1602 (JP); HASHINO, Akira, Kanonji-shi Kagawa 769-1602 (JP)
(74) Representative: Peter, Julian
(86) International application number: PCT/JP2009/071880
(87) International publication number: WO 2010/074339

(56) References cited:
- EP-A1- 1 669 046
- JP-A- 9 108 262
- JP-A- 2003 033 397
- JP-A- 2004 181 086
- JP-A- 2004 181 086
- JP-A- 2004 298 271
- JP-A- 2007 089 907
- US-A1- 2004 176 734
- US-A1- 2008 119 810

## Description

### TECHNICAL FIELD

The present invention relates to an absorbent article used for sanitary napkins, pantiliners and incontinence pads, etc., which is a relatively thin absorbent article, and which does not cause twisting and exhibiting excellent absorptivity even in use for a long time.

### BACKGROUND ART

In a sanitary napkin, compressed grooves are generally disposed for control of a desired deformation or for partial hardening to prevent generation of twisting (Japanese Unexamined Patent Publication (Kokai) No. 9-108262).

On the other hand, as for the sanitary napkin, a thin type is preferred. Some thin napkins, leakage-preventing grooves are disposed (Kokai No. 2002-65741). By disposing leakage-preventing grooves, fitness to the body may be enhanced, but in a thin napkin using materials with a small total basis weight or in a small quantity, there is a problem that the rigidity of the product is reduced, and therefore twisting (deformation of the absorbent article upon experience of a sideways force) occurs in use for a long time, as a result, menstrual blood leaks.

In order to solve such a problem, an absorption body obtained by joining an absorbent fiber and a highly absorbent polymer is generally used. Use of this absorption body is advantageous in that the physical properties are not greatly changed even in a wet state, but the cost rises (Kokai No. 2003-291234). On the other hand, when a stack of layers of fluffed pulp obtained by disintegrating a pulp sheet is processed into a thin absorption body, the cost is low, but the inside or surface of the absorption body is not bonded by a binder or the like, and therefore, when the absorption body is in a wet state or is applied with a pressure, twisting readily occurs.

### SUMMARY OF THE INVENTION

An object of the present invention is to provide an absorbent article which is a relatively thin absorbent article and which is hardly twisted while fitting to the body and exhibits excellent absorptivity.

Under these circumstances, the present inventors have made intensive studies, as a result, it has been found that when on the skin-contact surface side of the absorbent article, a compressed groove for integrating a liquid-permeable sheet and an absorption body is disposed at least in both longitudinal edge parts in the center region of the absorption body and a plurality of point-like compressed parts for integrating the liquid-permeable sheet and the absorption body are disposed at intervals, the above-described object can be attained. The present invention has been accomplished based on this finding.

That is,
(1) the present invention provides an absorbent article comprising at least a liquid-permeable sheet, a liquid-impermeable sheet, and an absorption body sandwiched between the liquid-permeable sheet and the liquid-impermeable sheet, wherein
   on the skin-contact surface side of the absorbent article, a compressed groove is disposed in both longitudinal edge parts at least in the center region of the absorption body so as to integrate the liquid-permeable sheet and the absorption body and a plurality of point-like compressed parts are disposed at intervals.
(2) The present invention provides the absorbent article according to (1), wherein the point-like compressed parts are provided at least in the longitudinal front or rear side with respect to the center region of the absorption body.
(3) The present invention provides the absorbent article according to (1) or (2), wherein the point-like compressed parts are provided at least in the region crossing a folding position at which the absorbent article is folded for packaging.
(4) The present invention provides the absorbent article according to any one of (1) to (3), wherein the absorption body is obtained by further wrapping an absorption body containing fluffed pulp in a paper backing.
(5) The present invention provides the absorbent article according to any one of (1) to (4), wherein the thickness of the absorption body is 8 mm or less_and the thickness of the absorption body in the point-like compressed part-disposed region is 50% or less of the thickness of the absorption body in the region where the point-like compressed parts are not disposed.
(6) The present invention provides the absorbent article according to any one of (1) to (5), wherein the compressed groove consists of a low-compressed part and a high-compressed part and the depth of the point-like compressed part is larger than the depth of the low-compressed part.
(7) The present invention provides the absorbent article according to any one of (1) to (6), wherein the width of the top-surface opening of the point-like compressed part is smaller than the maximum width of the top-surface opening of the compressed groove.
(8) The present invention provides the absorbent article according to any one of (1) to (7), wherein the absorption body has a higher basis weight in the center region of the absorption body than in the regions except for the center region and the point-like compressed parts are disposed such that in the vicinity of the center of the absorption body, the interval between point-like compressed parts becomes wider than in the regions on the front and rear sides with respect to the vicinity of the center.

The absorbent article of the present invention is relatively thin, nevertheless, is hardly twisted while fitting to the body and can efficiently absorb liquid excrement.

### BRIEF DESCRIPTION OF THE DRAWINGS

Fig. 1 is a plan view of an embodiment of a representative absorbent article of the present invention.
Fig. 2 shows a plan view illustrating one embodiment of the sanitary napkin of the present invention, and a cross-sectional view along A-A'.
Fig. 3 shows a plan view illustrating one embodiment of the sanitary napkin of the present invention, and a cross-sectional view along A-A'.
Fig. 4 shows a partially enlarged view of the A-A' cross-section of Fig. 3.
Fig. 5 shows a plan view illustrating one embodiment of the sanitary napkin of the present invention.

### MODE FOR CARRYING OUT THE INVENTION

The present invention is described below by referring to the drawings, but the present invention is not limited to those shown in the drawings.

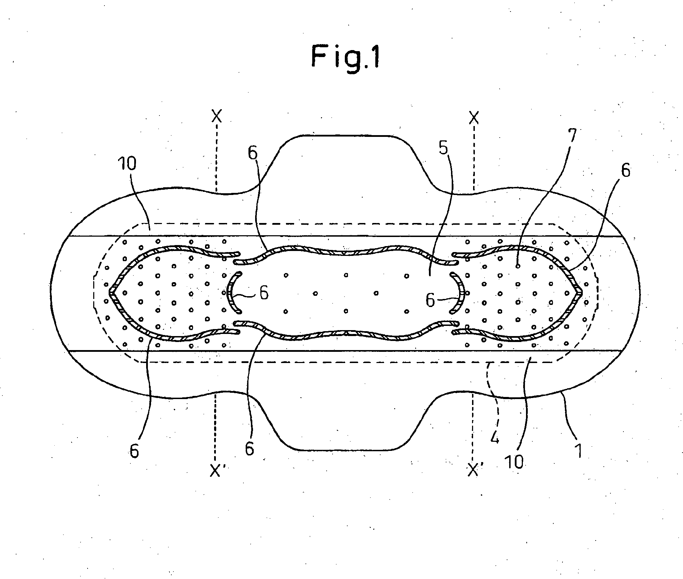

Fig. 1 is a plan view of an embodiment of a typical absorbent article of the present invention.

As shown in Fig. 1, the absorbent article 1 of the first embodiment comprises at least a liquid-permeable sheet 2, a liquid-impermeable sheet 3, and an absorption body 4 sandwiched between the liquid-permeable sheet and the liquid-impermeable sheet.

On the skin-contact surface side of the absorbent article of the present invention, compressed grooves 6 for integrating the liquid-permeable sheet and the absorption body are disposed at least in both longitudinal side edge parts of the center region 5 of the adsorption body. The compressed groove fulfills a role of not only integrating the liquid-permeable sheet and the absorption body but also stopping lateral spreading of a liquid excrement from the body, such as menstrual blood and urine, and allowing the liquid excrement to be efficiently transferred beneath the absorption body. As shown in Fig. 1, the compressed groove does not need to be necessarily disposed in a continuous manner, but is preferably disposed to define a nearly closed form and surround the center region of the absorption body. The center region of the absorption body is a region where the excrement is mostly absorbed, and even it the excrement is diffused in the width direction in the center part of the absorption layer, the flow of the excrement can be guided to the center region of the absorption layer along the compressed groove and the leakage of the excrement can be suppressed. The compressed groove preferably consists of a high-compressed part compressed in a ratio of 50% or more based on the thickness of the absorption body and a low-compressed part compressed in a ratio of 20% or more. By providing a high-compressed part and a low-compressed part, the joining force with the absorption body can be enhanced and diffusion of an excrement can be prevented by collecting the excrement in the high-compressed part.

The shape of the high-compressed part is not particularly limited and, for example, may be in planar view, a heart shape, a diamond shape, a box shape, a triangular shape, a dot shape or a star shape. In the case of having a pattern of hearts, stars or the like, discomfort during menstruation may be relieved by its design effect. The high-compressed part and the low-compressed part does not need to be necessarily disposed as long as they are continuously disposed in the longitudinal direction and may be disposed, for example, in a grid-like pattern.

The width of the top-surface opening of the compressed groove is from 1 to 10 mm, preferably from 1.5 to 5 mm, and is usually about 2.0 mm. If the width is less than 1 mm, sufficient rigidity is not obtained and the absorbent article cannot be controlled so as to become the desired deformation, whereas if it exceeds 10 mm, a portion being highly rigid and coming into contact with the skin is increased to give an uncomfortable feeling in use. Incidentally, when the width of the top-surface opening of the compressed groove varies depending on the position of the compressed groove, the width of the top-surface opening means the maximum width of top-surface openings.

On the skin-contact surface side of the absorbent article, a plurality of point-like compressed parts 7 for integrating the permeable sheet and the absorption body are further disposed at constant intervals. The point-like compressed part is preferably disposed in a region longitudinally forward or rearward (preferably rearward) rather than the center region of the absorption body. The forward or rearward region is susceptible to motion of the body during, such as waking or standing/sitting, but by providing a point-like compressed part in addition to the compressed groove, the movement of the material constituting the absorption body can be controlled, and the material is allowed to uniformly exist in the entire absorbent article, thereby allowing for little twisting. As a result, despite the same basis weight, the rigidity of the absorbent article is higher than that of the absorbent article where only a compressed groove or only a point-like compressed part is provided. In the following, unless otherwise indicated, the "rigidity" means the rigidity of the entire absorbent article.

Also, by providing a point-like compressed part 7, the contact area with the skin can be decreased, so that the absorbent article can be prevented from sticking to the body, and stuffiness can be reduced. The point-liked compressed part is preferably disposed at least in the region extending across the folding position for packaging (for example, X-X' in Fig. 1) of the absorbent article. Usually, the absorbent article is individually packaged in a state of being folded, and therefore the material constituting the absorption body in the portion corresponding to the folding position is liable to move in use and readily causes twisting. By disposing point-like compressed parts to spread across the folding position for packaging, the movement of the material constituting the absorption body at the folding position for packaging can be controlled, and therefore the absorption body is hardly twisted.

Optionally, point-like compressed parts may be disposed almost over the entire surface of the absorption body, including the center region of the absorption body. By arrangement over the entire surface, the movement of the material constituting the absorption body can be more successfully controlled and in turn, occurrence of twisting is more reduced.

By the arrangement of point-like compressed parts, not only twisting is reduced or eliminated but also a liquid excrement flowing on the surface of the absorbent article is easily caught. That is, by disposing point-like compressed parts, a high-density portion (a portion where a point-like compressed part is disposed) and a low-density portion (a portion where a point-like compressed part is not disposed) having more spaces than the high-density portion are formed in the absorption body, where the low-density portion is excellent in instantaneous absorption for an excrement discharged in a large amount at a time and the high-density portion can attract the absorbed excrement and give an ability to spare for absorption to the low-density portion.

The width of the top-surface opening of the point-like compressed part is from 0.3 to 5 mm, preferably from 0.5 to 3 mm, and is usually about 1.2 mm. If the width of the top-surface opening of the point-like compressed part is less than 0.3 mm, only a hole is created and the part cannot serves as a compressed part for increasing the rigidity, failing in preventing twisting. If the width exceeds 5 mm, a rigid portion brought into contact with the skin is increased to give an uncomfortable feeling in use. Incidentally, in the case where the shape of the top-surface opening of the point-like compressed part is polygonal, the diameter of the inscribed circle is defined as the width of the top-surface opening. The shape of the point-like compressed part is not particularly limited and may be, for example, in planar view, a heart shape, a diamond shape, a box shape, a triangular shape, a dot shape or a star shape. In the case of having a pattern of hearts, stars or the like, discomfort during menstruation may be relieved by its design effect.

The distance between a compressed groove and a point-like compressed part and the distance between point-like compressed parts are 20 mm or less, preferably 15 mm or less, more preferably 10 mm or less. If the distance between compressed parts exceeds 20 mm, the rigidity between compressed parts is decreased and the absorption body may be twisted starting therefrom. Also, the width of the top-surface opening of the point-like compressed part is preferably smaller than the width of the top-surface opening of the compressed groove. If the width is larger than the width of the top-surface opening of the compressed groove, the point-like compressed part surpasses in the rigidity and may inhibit the compressed groove behavior of controlling the shape of the absorption body to fit to the body when a force is laterally imposed. The point-like compressed parts are preferably formed to spread across the compressed groove. In the vicinity of the compressed groove, the absorption body is liable to be greatly twisted, and the point-like compressed part is provided so as to prevent pulp or fiber constituting the absorption body from being transferred more than necessary, while ensuring deformation to follow the movement of the body.

The depth of the point-like compressed part is preferably larger than the depth of the low-compressed part of the compressed groove. If the depth is smaller than the depth of the low-compressed part, the effect of integrating the liquid-permeable sheet and the absorption body is not obtained by the point-like compressed part. On the other hand, if it is larger than the depth of the high-compressed part of the compressed groove, the rigidity becomes excessively strong and may inhibit behavior of the compressed groove.

For the absorption body, a powder or granular material formed from a mixture obtained by pulverizing sheet-like wood pulp and mixing the resulting fluffed pulp with a highly absorptive polymer is used. In place of wood pulp, for example, chemical pulp, cellulose fiber or artificial cellulose fiber such as rayon and acetate may be used. Examples of the highly absorptive polymer include a starch-based, acrylic acid-based or amino acid-based, particulate or fibrous polymer. To this powder or granular material, a thermal fusion bonding agent for thermally fusing the powder or granular material may be further added.

The absorption body is usually wrapped with a paper backing 8 such as tissue paper. The thickness of the absorption body is 8 mm or less, preferably 5 mm or less. If the thickness of the absorption body exceeds 8 mm, since point-like compressed parts are disposed at intervals, the liquid-permeable sheet cannot be elongated when forming the point-like compressed part and is ruptured, failing in forming the compressed part. Even if the compressed part can be formed, the absorbent article becomes hard and bad usefulness results. The thickness of the absorption body in the region where point-like compressed parts are disposed is preferably 50% or less of the thickness of the absorption body in the region where the point-like compressed part is not disposed. If the thickness exceeds 50%, adequate rigidity is not obtained.

The liquid-permeable sheet is entirely or partially liquid-permeable, and the liquid permeation area is formed of, for example, a resin film having formed therein a large number of liquid permeation holes, a net-like sheet having a large number of meshes, a liquid-permeable nonwoven fabric or a woven fabric. As for the resin film or net-like sheet, those formed of polypropylene, polyethylene, polyethylene terephthalate or the like may be used. As for the nonwoven fabric, for example, a spunlaced nonwoven fiber formed of a cellulose fiber (e.g., rayon), a synthetic resin fiber or the like, and an air-through nonwoven fabric formed of the synthetic resin fiber above, may be used. The basis weight of the liquid-permeable sheet is preferably from 15 to 100 g/m², more preferably from 20 to 50 g/m², still more preferably from 10 to 40 g/m². If the basis weight is less than 15 g/m², an adequate surface strength is not obtained and the sheet may be ruptured during use. If it exceeds 100 g/m², excessive roughness is produced to give an uncomfortable feeling during use. The density is 0.12 g/cm² or less and is not particularly limited, as long as the sheet is liquid-permeable. If the density exceeds the value above, smooth permeation between liquid-permeable fibers is difficult. In the case where the excrement is menstrual blood, a sheet with a low density is preferred, because the menstrual blood is viscous compared to urine and the like.

The liquid-impermeable sheet is sparingly permeable to liquid and does not pass a liquid excrement. Specifically, the liquid-impermeable sheet includes a conventionally employed material, such as film mainly composed of a low-density polyethylene resin and prepared to have a basis weight of 15 to 30 g/m².

On the non-skin-contact surface of the liquid-impermeable sheet, a slip stopper such as adhesive for fixing to an underwear such as panty is preferably provided on the non-skin-contact surface side of the liquid-impermeable sheet. The point-like compressed part is preferably disposed at a position where the slip stopper is not provided in the thickness direction. This is because of the following reasons. Usually, the liquid-impermeable sheet is fixed to the absorption body by an adhesive or the like, and therefore the absorption body in the portion corresponding to the position at which a slip stopper is provided on the liquid-impermeable sheet can move together with a panty even when the crotch portion of the panty moves in response to the movement of the body. On the other hand, the absorption body in the portion corresponding to the position at which a slip stopper is not provided on the liquid-impermeable sheet may allow for creation of a space between the panty and the liquid-impermeable sheet when the crotch portion of the panty moves, and movement of the material constituting the absorption body corresponding to that portion cannot be controlled, as a result, twisting is readily caused. Examples of the slip stopper include a hot-melt adhesive. The slip stopper may be continuously coated in a line or belt fashion or may be coated intermittently. For the reason above, even when there is a portion where a slip stopper is intermittently provided, the surface profile of the absorbent article is easily maintained thanks to the arrangement of point-like compressed parts.

In the absorption body and/or the liquid-impermeable sheet, a colored region may be disposed. The colored region may be partially disposed, and in such a case, the colored region needs to be disposed in the region where the point-like compressed part is provided.

When the colored region is disposed, in the point-like compressed part, the color of the colored region is seen through, because the thickness of the absorption body is reduced therein. Also, the position of the point-like compressed part can be clearly recognized with an eye before use, and this is likely to give a feeling of security for absorptive power and an impression of good air permeability to the wearer. Also, during use or after use, the visibility of the liquid excrement absorbed differs between the position of the point-like compressed part and other positions, and the wearer readily realizes the absorptive power.

The colored region needs to be in a color different from the absorption body or liquid-impermeable sheet where the colored region is provided. The color may be selected, for example, by taking into consideration the psychological effect, but any color may be used. The material constituting the colored region is not particularly limited as long as it is a colored material such as a resin in which ink, a coating material or a colorant is contained, a nonwoven fabric in which a colorant is mixed, a nonwoven fabric whose surface is coated with a colorant, a film in which a colorant is mixed, and a film whose surface is coated with a colorant, but a hot-melt adhesive containing a colorant is preferred. For example, between the liquid-permeable sheet and the absorption body or between the absorption body and the liquid-impermeable sheet, a hot-melt adhesive containing a colorant may be coated to join respective members or may be disposed after forming it into a film or a sheet. Also when a colored liquid-impermeable sheet is provided, the color of the liquid-impermeable sheet can be viewed through the point-like compressed part.

The liquid-permeable sheet 2 and the absorption body 4 are preferably further integrated by an adhesive layer. When integrated by an adhesive layer, twisting of the absorbent article can be more reduced. Examples of the adhesive layer include a layer composed of a hot-melt resin or a hot-melt adhesive. After providing the adhesive layer, heat embossing is applied to form the compressed groove and the point-like compressed part, whereby not only the joining force between the liquid-permeable sheet and the absorption body can be enhanced but also the strength or durability of the stack of these members can be increased and in turn, twisting can be more reduced. If desired, a hydrophilic fiber-containing nonwoven fabric may be inserted as a second sheet between the liquid-permeable sheet and the absorption body.

In the widthwise edge part of the absorbent article, a permeable side sheet 10 joined at least partially to both the right and left sides of the liquid-permeable sheet 2 may be disposed.

Also, in the edge part on both sides near the center of the absorption body, a wing part may be formed by letting the side sheet and the liquid-impermeable sheet 3 stick out outwardly in the width direction.

The absorbent article of the present invention can be used as a sanitary napkin, a pantiliner, an incontinence pad or the like.

The production method of the absorbent article of the present invention includes at least an embossing step of stacking a liquid-permeable sheet 2 and an absorption body 4 and compressing the stack in the thickness direction. The compressed groove and the point-like compressed part may be simultaneously formed by the embossing or may be separately formed in any order. For example, in the case of simultaneously forming these grooves and parts, between a roll (first roll) having on the roller surface thereof convex streaks corresponding to the entire shape of the compressed groove and convex parts corresponding to the entire shape of the point-like compressed part and a smooth roll (second roll), an absorbent material after stacking a liquid-permeable sheet and an absorption body is disposed by arranging the liquid-permeable sheet to come into contact with the first roll, and the absorbent material is compressed. An adhesive such as hot-melt adhesive may be applied between the liquid-permeable sheet and the absorption body. Incidentally, in the absorbent article of the present invention, the compressed groove and the point-like compressed part are formed by embossing, and therefore even when an adhesive is not used, the liquid-permeable sheet and the absorption body can be integrated. Also, even if an adhesive is used, the amount used thereof can be decreased and production at a low cost can be achieved.

Finally, a liquid-impermeable sheet 3 is provided to the non-skin-contact surface side of the absorption body 4 of the embossed absorbent material, and the absorption body 4 and the liquid-impermeable sheet 3 are joined. The absorption body and the liquid-impermeable sheet may be joined by using, for example, a hot-melt adhesive.

In the embodiment above, the production method of a sanitary napkin is described as an example, but this method can be also applied to the production of other absorbent articles, for example, a pantiliner or an incontinence pad.

### EXAMPLES

The present invention is described in greater detail below, but the present invention is not limited to these Examples.

### Example 1

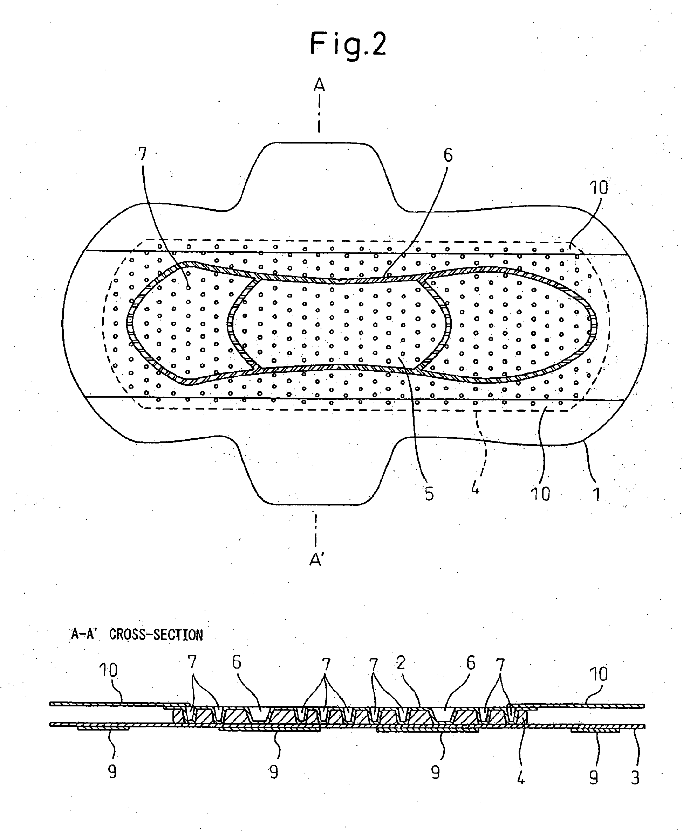

Fig. 2 shows a plan view illustrating one embodiment of the sanitary napkin of the present invention, and a cross-sectional view along A-A'. In Fig. 2, a compressed groove 6 is disposed in the center region 5 of the absorption body and the longitudinally forward and rearward parts of the center region to continue at the center region and at the same time, point-like compressed parts 7 are disposed at the equal intervals almost over the entire surface of the absorption body 4. The sanitary napkin of Fig. 2 was produced as follows.

First, the following members were prepared.

### (1) Absorption Body 4

Fluffed pulp (basis weight: 180 g/m²) and a highly absorptive polymer (basis weight: 30 g/m²) were almost uniformly dispersed, and the dispersion was wrapped with a tissue paper (basis weight: 15 g/m²), and further, almost the entire surface was pressed to give a thickness of about 2 mm. The absorption body after pressing had a length of 195 mm and a width of 75 mm.

### (2) Liquid-Permeable Sheet 2

An air-through nonwoven fabric (basis weight: 30 g/m², length: 230 mm, width: 80 mm) was used as the liquid-permeable sheet.

### (3) Second Sheet (not shown)

An air-through nonwoven fabric (basis weight: 30 g/m², length: 230 mm, width: 80 mm) was used as the second sheet

### (4) Liquid-Impermeable Sheet 3

A film containing a colorant was used.

The absorption body 4, the second sheet and the liquid-permeable sheet 2 were stacked in this order, a hot-melt adhesive was coated between respective layers, and heat embossing was applied using a heat embossing roll to integrate the liquid-permeable sheet, the second sheet and the absorption body.

By the heat embossing above, a compressed groove 6 and point-like compressed parts 7 were formed at the positions shown in Fig. 2. The width of the compressed groove was set to 2.0 mm. The point-like compressed part was formed by compression in a ratio of 50 to 99% based on the thickness of the absorption body. The diameter of the point-like compressed part was set to 1.2 mm, and the distance between point-like compressed parts was set to 10 mm.

A side sheet 10 was disposed in the widthwise edge part of the integrated laminate, and a liquid-impermeable sheet 3 was disposed on the non-skin-contact surface side of the laminate. Furthermore, the side sheet and the liquid-impermeable sheet was laminated together near the absorption body in the longitudinal direction, and the laminate was cut into a product shape. Incidentally, on the non-skin-contact surface side of the liquid-impermeable sheet and the wing part, a slip stopper 9 (hot-melt adhesive) for fixing to a panty was applied.

In the sanitary napkin of this Example, point-like compressed parts are disposed almost over the entire surface of the absorption body and therefore, twisting is hardly caused.

### Example 2

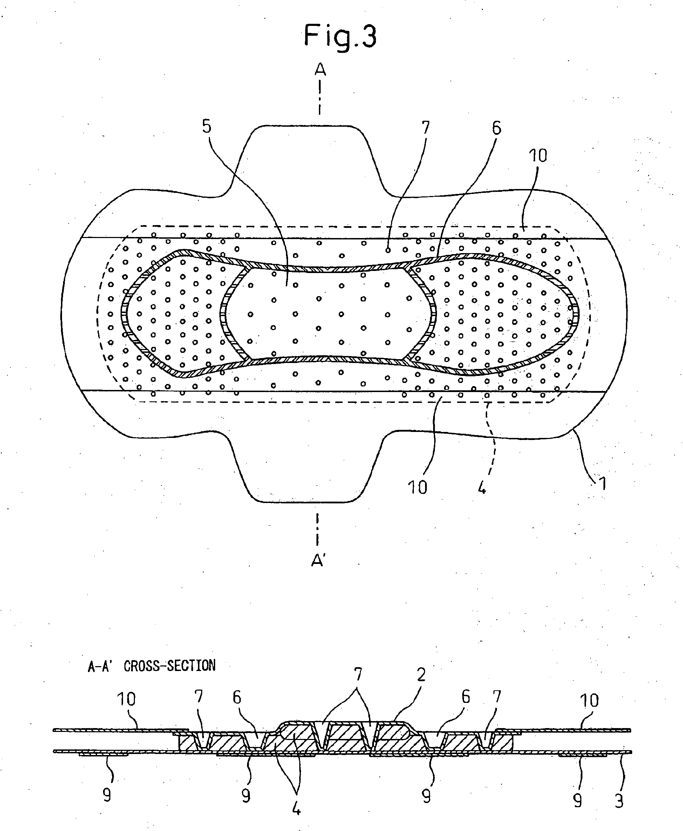

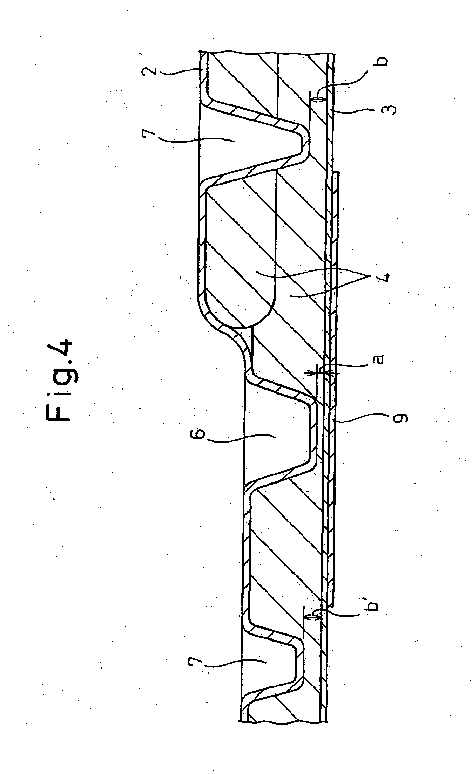

Fig. 3 shows a plan view illustrating one embodiment of the sanitary napkin of the present invention, and a cross-sectional view along A-A'. In Fig. 3, a compressed groove 6 is disposed in the center region 5 of the absorption body and the longitudinally forward and rearward parts of the center region to continue at the center region. Also, point-like compressed parts 7 are disposed almost over the entire surface of the absorption body 4 at such intervals as becoming sparse near the center of the absorption body and dense in the forward and rearward regions near the center. Fig. 4 shows an example of the partially enlarged view of the A-A' cross-section of Fig. 3. In Fig. 4, the thickness (that is, the distance b) of the portion having a point-like compressed part 7 near the center of the absorption body and the thickness (that is, the distance b') of the portion having a point-like compressed part 7 in the forward and rearward regions near the center are the same and the depth of the point-like compressed part is smaller than the depth (that is, the distance a) of the high-compressed part of the compressed groove 6.

The members used for producing the sanitary napkin of this Example are the same as in Example 1 except for the absorption body. In the absorption body used in this Example, the basis weight in the center region of the absorption body was 260 g/m², the basis weight in the region other than the center region was 130 g/m², the length was 195 mm, and the width was 75 mm.

Heat embossing was performed in the same manner as in Example 1 to form a compressed groove 6 and point-like compressed part 7 at the positions shown in Fig. 3. The width of the compressed groove was set to 2.0 mm. The diameter of the point-like compressed part was set to 1.2 mm, the distance between point-like compressed parts was set to 10 mm near the center of the absorption body and 5 mm in the forward and rearward regions near the center.

In the sanitary napkin of this Example, the basis weight is high in the center region of the absorption body, where a liquid excrement is directly absorbed, but point-like compressed parts are disposed near the center of the absorption body, whereby twisting is hardly caused. Furthermore, point-like compressed parts are sparsely disposed near the center of the absorption body to increase the instantaneous absorption, and point-like compressed parts are densely disposed in the forward and rearward regions near the center of the absorption body to reduce the contact area with the skin, whereby the absorbent article can be made to hardly stick to the body.

### Example 3

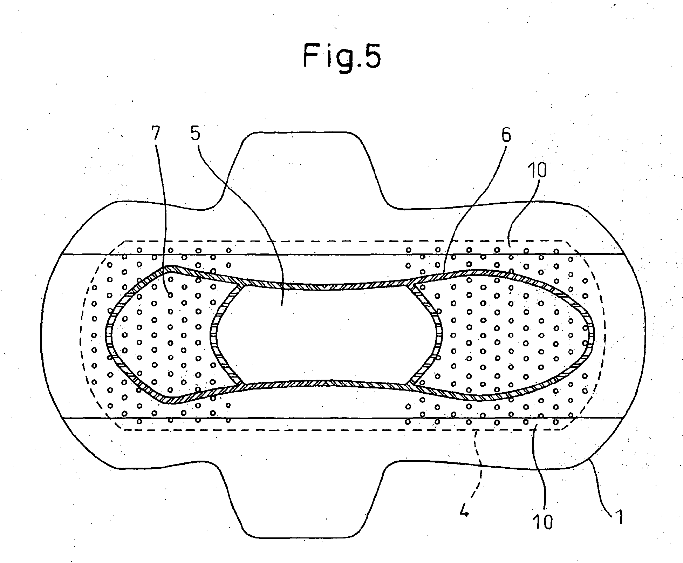

Fig. 5 shows a plan view illustrating one embodiment of the sanitary napkin of the present invention. In Fig. 5, a compressed groove 6 is disposed in the center region 5 of the absorption body and the longitudinally forward and rearward parts of the center region to continue at the center region, and point-like compressed parts 7 are disposed at the equal intervals only in the forward and rearward regions near the center of the absorption body.

All of the members used for producing the sanitary napkin of this Example are the same as in Example 2.

Heat embossing was performed in the same manner as in Example 1 to form a compressed groove 6 and point-like compressed part 7 at the positions shown in Fig. 5. The point-like compressed part was formed by compression in a ratio of 50 to 99% based on the thickness of the absorption body. The width of the compressed groove was set to 2.0 mm. The diameter of the point-like compressed part was set to 1.2 mm, and the distance between point-like compressed parts was set to 8 mm.

In the sanitary napkin of this Example, due to a high basis weight in the center region of the absorption body, the contact ratio with the skin is high. In the forward and rearward regions near the center of the absorption body, twisting is less caused than in the center region of the absorption body. However, since utterly no point-like compressed part is disposed near the center of the absorption body, separation between the liquid-permeable sheet and the absorption body may be separated in use compared with the sanitary napkin of Example 2.

As another embodiment of the absorbent article of the present invention, at the time of providing point-like compressed parts, the compressed parts may be provided also on the liquid-impermeable sheet side by using a second roll having provided thereon convex parts and compressing the absorption body from the liquid-impermeable sheet side. Particularly, in the center region where the basis weight or thickness of the absorption body is large, when a smooth second roll is used, the distance for which the absorption body is pressed by the convex part of the first roll becomes large and the surface sheet in the portion abutting with the convex part may be ruptured. However, when the absorption body is compressed also from the liquid-impermeable sheet side, the distance for which the absorption body is pressed from one side becomes short thanks to pressing by respective convex parts of upper and lower rolls and rupture of the sheet hardly occurs.

The absorbent article of the present invention may be in an embodiment where when the absorbent article is viewed from the liquid-impermeable sheet side, light passes and is seen through the high-compressed part and the point-like compressed part. This is achieved by the formation to a larger depth of the point-like compressed part than the low-compressed part of the compressed groove or because of the magnitude or the like of the width of the top-surface opening of the point-like compressed part or the high-compressed part. In turn, before use, the position of the point-like compressed part and the position of the high-compressed part can be clearly recognize with an eye by the wearer and an impression of good absorptive power and air permeability can be given. Furthermore, although it may vary depending on the magnitude of the top-surface opening width of the point-like compressed part or the high-compressed part, when such a part is formed in a pattern of hearts, stars or the like, the pattern can be clearly recognized with an eye also from the liquid-impermeable sheet side, and discomfort during menstruation can be relived thanks to its design effect.

### DESCRIPTION OF REFERENCE NUMERALS

- 1:: Absorbent article
- 2:: Liquid-permeable sheet
- 3:: Liquid-impermeable sheet
- 4:: Absorption body
- 5:: Center region of absorption body
- 6:: Compressed groove
- 7:: Point-like compressed part
- 8:: Paper backing
- 9:: Slip stopper
- 10:: Side sheet

## Claims

1. An absorbent article comprising at least a liquid-permeable sheet (2), a liquid-impermeable sheet (3), and an absorption body (4) sandwiched between said liquid-permeable sheet and said liquid-impermeable sheet, wherein
on the skin-contact surface side of said absorbent article (1), a compressed groove (6) is disposed in both longitudinal edge parts at least in the center region of the absorption body (4) so as to integrate said liquid-permeable sheet (2) and said absorption body (4), and a plurality of point-like compressed parts (7) are disposed at intervals so as to integrate said liquid permeable sheet (2) and said absorption body (4),
wherein said compressed groove (6) consists of a low-compressed part and a high-compressed part and the depth of said point-like compressed parts (7) is larger than the depth of said low-compressed part,
wherein the width of the top-surface opening of said point-like compressed parts (7) is smaller than the maximum width of the top-surface opening of said compressed groove (6).

2. The absorbent article according to claim 1, wherein said point-like compressed parts (7) are provided at least in the longitudinal front or rear side with respect to the center region of the absorption body (4).

3. The absorbent article according to claim 1 or 2, wherein said point-like compressed parts (7) are provided in the center region of said absorption body (4) and in the region crossing a folding position at which the absorbent article (1) is folded for packaging.

4. The absorbent article according to any one of claims 1 to 3, wherein said absorption body (4) is obtained by further wrapping an absorption body containing fluffed pulp in a paper backing.

5. The absorbent article according to any one of claims 1 to 4, wherein the thickness of said absorption body (4) is 8 mm or less and the thickness of the absorption body (4) in the point-like compressed part-disposed region is 50% or less of the thickness of the absorption body (4) in the region where said point-like compressed parts (7) are not disposed.

6. The absorbent article according to any one of claims 1 to 5, wherein said absorption body (4) has a higher basis weight in the center region of the absorption body than in the regions except for said center region and the point-like compressed parts (7) are disposed such that in the vicinity of the center of the absorption body (4), the interval between point-like compressed parts (7) becomes wider than in the regions on the front and rear sides with respect to said vicinity of the center.

7. The absorbent article according to any one of claims 1 to 6, wherein said point-like compressed parts (7) are formed to spread across said compressed groove (6).

8. The absorbent article according to any one of claims 1 to 7, wherein said compressed groove (6) is disposed to define a nearly closed form and surround said center region of the absorption body (4).

9. The absorbent article according to any one of claims 1 to 8, wherein said high-compressed part and said low-compressed part does are continuously disposed in the longitudinal direction.

## Patentansprüche

1. Absorbierender Artikel mit mindestens einer flüssigkeitsdurchlässigen Lage (2), einer flüssigkeitsundurchlässigen Lage (3) und einem Absorptionskörper (4), der zwischen die flüssigkeitsdurchlässige Lage und die flüssigkeitsundurchlässige Lage eingelegt ist, wobei
auf der Hautkontaktseite des absorbierenden Artikels (1) eine komprimierte Rinne (6) in beiden longitudinalen Randteilen zumindest in dem Zentralbereich des Absorptionskörpers (4) vorgesehen ist, um die flüssigkeitsdurchlässige Lage (2) und den Absorptionskörper (4) zu integrieren, und eine Vielzahl von punktartigen komprimierten Teilen (7) in Abständen angeordnet ist, um die flüssigkeitsdurchlässige Lage (2) und den Absorptionskörper (4) zu integrieren,
wobei die komprimierte Rinne (6) aus einem gering-komprimierten Teil und einem hoch-komprimierten Teil besteht und die Tiefe der punktartigen komprimierten Teile (7) größer ist als die Tiefe des gering-komprimierten Teils, wobei die Breite der Oberflächen-Öffnung der punktartigen komprimierten Teile (7) kleiner ist als die maximale Breite der Oberflächen-Öffnung der komprimierten Rinne (6).

2. Absorbierender Artikel nach Anspruch 1, wobei die punktartigen komprimierten Teile (7) zumindest auf der longitudinalen Vorder- oder Rückseite bezüglich des Zentralbereichs des Absorptionskörpers (4) vorgesehen sind.

3. Absorbierender Artikel nach Anspruch 1 oder 2, wobei die punktartigen kornprimierten Teile (7) in dem Zentralbereich des Absorptionskörpers (4) und in dem eine Faltposition, in dem der absorbierende Artikel (1) zum Verpacken gefaltet wird, kreuzenden Bereich vorgesehen sind.

4. Absorbierender Artikel nach einem beliebigen der Ansprüche 1 bis 3, wobei der Absorptionskörpers (4) durch weiteres Umhüllen eines Flockenzellstoff enthaltenden Absorptionskörpers in eine Papierhülle erhalten wird.

5. Absorbierender Artikel nach einem beliebigen der Ansprüche 1 bis 4, wobei die Dicke des Absorptionskörpers (4) 8mm oder weniger ist und die Dicke des Absorptionskörpers (4) in den Bereichen der punktartigen komprimierten Teilen (7) 50% oder weniger ist als die Dicke des Absorptionskörpers (4) in dem Bereich, in dem die punktartigen komprimierten Teile (7) nicht vorgesehen sind.

6. Absorbierender Artikel nach einem beliebigen der Ansprüche 1 bis 5, wobei der Absorptionskörper (4) in dem Zentralbereich des Absorptionskörpers ein größeres Basisgewicht aufweist als in den anderen Bereichen als dem Zentralbereich und die punktartigen komprimierten Teile (7) derart angeordnet sind, dass in der Mitte des Absorptionskörpers (4) der Abstand zwischen den punktartigen komprimierten Teilen (7) größer wird als in den Bereichen auf den Vorder- und Rückseiten zu der Nachbarschaft der Mitte.

7. Absorbierender Artikel nach einem beliebigen der Ansprüche 1 bis 6, wobei die punktartigen komprimierten Teile (7) gebildet sind, sich über die komprimierte Rinne (6) zu verteilen.

8. Absorbierender Artikel nach einem beliebigen der Ansprüche 1 bis 7, wobei die komprimierte Rinne (6) angeordnet ist, eine fast geschlossene Form aufzuweisen und den Zentralbereich des Absorptionskörpers (4) zu umgeben.

9. Absorbierender Artikel nach einem beliebigen der Ansprüche 1 bis 8, wobei der hoch-komprimierte Teil und der gering-komprimierte Teil kontinuierlich in der Längsrichtung angeordnet sind.

## Revendications

1. Article absorbant comprenant au moins une feuille perméable aux liquides (2), une feuille imperméable aux liquides (3) et un corps d'absorbant (4) qui est en sandwich entre la feuille perméable aux liquides et la feuille imperméable aux liquides, dans lequel
une rainure comprimée (6) dans les deux parties de bord longitudinal est au moins prévue dans la région centrale du corps d'absorption (4) sur le côté en contact avec la peau de l'article absorbant (1) pour intégrer la feuille perméable aux liquides (2) et le corps d'absorption (4), et une pluralité des parties comprimées ponctuelle (7) disposées à des intervalles afin d'intégrer la feuille perméable aux liquides (2) et le corps d'absorption (4),
dans lequel la rainure comprimée (6) se compose d'une partie comprimée faible et une partie comprimée fortement et la profondeur des parties comprimées ponctuelle (7) est supérieure à la profondeur de la partie comprimée faible,
dans lequel la largeur de l'ouverture à la surface des parties comprimées ponctuelle (7) est plus petite que la largeur maximale de l'ouverture à la surface de la rainure comprimée (6).

2. Article absorbant selon la revendication 1, dans lequel les parties comprimées ponctuelle (7) sont prévus au moins sur la surface longitudinale avant et derrière par rapport à la région centrale du corps d'absorption (4).

3. Article absorbant selon les revendications 1 ou 2, dans lequel les parties comprimées ponctuelles (7) sont prévues dans la région centrale du corps d'absorption (4) et dans la zone d'intersection dans une position de pliage, dans lequel l'article absorbant (1) est plié pour l'emballage.

4. Article absorbant selon l'une quelconque des revendications 1 à 3, dans lequel le corps absorbant (4) est obtenue en enroulant en outre le corps absorbant contenant une pâte défibrée dans une enveloppe de papier.

5. Article absorbant selon l'une quelconque des revendications 1 à 4, dans lequel l'épaisseur du corps absorbant (4) est de 8 mm ou moins, et l'épaisseur du corps absorbant (4) dans les zones des parties comprimées ponctuelles (7) est 50% ou moins de l'épaisseur du corps d'absorption (4) dans la zone dans laquelle les parties comprimées ponctuelles (7) ne sont pas fournis.

6. Article absorbant selon l'une quelconque des revendications 1 à 5, dans lequel le corps (4) d'absorption ayant une poids de base dans la région centrale du corps absorbant plus grande que dans les autres régions différant à la région centrale et les parties comprimées ponctuelles (7) sont disposés de telle sorte dans la zone centrale du corps d'absorption (4) que la distance entre les parties comprimées ponctuelles (7) est plus grande que dans les régions sur les surfaces avant et arrière au voisinage du zone centrale.

7. Article absorbant selon l'une quelconque des revendications 1 à 6, dans lequel les parties comprimées ponctuelles (7) sont formées sur la rainure comprimé (6).

8. Article absorbant selon l'une quelconque des revendications 1 à 7, dans lequel la rainure comprimé (6) est agencé de façon à avoir une forme presque fermé pour l'entourer la zone centrale du corps absorbant (4).

9. Article absorbant selon l'une quelconque des revendications 1 à 8, dans lequel la partie comprimée fortement et la partie comprimée faible sont disposées en continu dans la direction longitudinal.
